# EUROPEAN PATENT APPLICATION

(11) **EP 2 286 764 A1**
(43) Date of publication of application: **23.02.2011**
(21) Application number: 10172668.5
(22) Date of filing: 12.08.2010
(51) Int. Cl.: A61F 2/16

(54) **Cassette for receiving an intraocular lens, lens injector device and method for advancing an intraocular lens out of a cassette**

(30) Priority: 18.08.2009 GB 0915099
(71) Applicant: Carl Zeiss Meditec SAS, 17053 La Rochelle Cedex 9 (FR)
(72) Inventor: Pankin, Dmitry, 81737, München (DE); Raquin, Vincent, 17000, La Rochelle (FR); Rathert, Brian, 17000, La Rochelle (FR)
(74) Representative: Lechner, Armin Anton

(57) **Abstract**

The invention relates to a cassette for receiving an intraocular lens (11), wherein the cassette (8, 8') is formed for inserting into a lens injector device (1) and has an entry region (12) for a plunger (4) of the injector device (1) and an exit region (15) for the lens (11), wherein the cassette (8, 8') has a bending element (34, 34') movably disposed on the cassette (8, 8') and formed for transferring a haptic part (17, 18) of the intraocular lens (11) loaded in the cassette (8, 8') from a rest-position (RP) into a bending position (BP). The invention also relates to a lens injector device (1) including a cassette (8, 8') as well as to a method for advancing an intraocular lens (11) out of a cassette (8, 8').

## Description

Cassette for receiving an intraocular lens, lens injector device and method for advancing an intraocular lens out of a cassette.

### Technical field

The invention relates to a cassette for containing an intraocular lens, wherein the cassette is formed for inserting into an injector device, wherein the injector device is formed for introducing the intraocular lens into the eye. The cassette has an entry region for the plunger of the injector device and an exit region for the lens. Moreover, the invention relates to an injector device with such a cassette. Furthermore, the invention relates to a method for storing an intraocular lens in a cassette as well as a method for advancing an intraocular lens out of a cassette.

### Prior art

An intraocular lens is an eye implant, which is inserted into the eye if the original natural lens has to be replaced due to the produced defective vision. In such cataract surgeries, through a small incision in the eye, the natural lens is fragmented and sucked off. Through such small incisions in the eye, then, the intraocular lens is inserted. Such an intraocular lens is known in manifold configuration and usually has an optic part and plural haptic parts. The intraocular lens is positionally stably held in the eye by the haptic parts.
For introducing such intraocular lenses into the eye, an injector device is used, wherein, here too, various configurations are known. From US 2005/0125000 A1, such an injector device is known. It has a plunger, which can be movably displaced in a tube. A cassette receiving the intraocular lens can be inserted into this tube. The plunger engages the cassette through an entry region and engages the optic part of the lens, which is disposed in horizontal direction in the cassette. Then, by means of the plunger, the lens is advanced out of the cassette through an exit region and advanced into a tapering injection channel following the tube of the injector device. There, the lens is automatically rolled up.

### Presentation of the invention

It is the object of the present invention to provide a cassette and an injector device as well as a method for storing an intraocular lens in a cassette and a method for advancing an intraocular lens out of a cassette, by which or in which damage of the intraocular lens, in particular of the haptic part, can be prevented. In particular, undesired mechanic stresses and force effects on the lens also are to be avoided.

This object is solved by a cassette having the features according to claim 1, an injector device having the features according to claim 13, and furthermore by a method having the features according to claim 16.

A cassette according to the invention is formed for receiving an intraocular lens, wherein the cassette is formed for inserting into a lens injector device and has an entry region for a plunger of the injector device and an exit region for the lens. The cassette includes at least one bending element disposed movably on the cassette and formed for transferring a haptic part of the intraocular lens loaded in the cassette from a rest-position into a bending position. By this configuration, it can be achieved that upon advancing the lens out of the cassette, undesired mechanical contacts of the plunger with specific regions of the lens are avoided. In particular, specific regions of the haptic part are therefore not loaded with undesired mechanic force effects such that the haptic part is taken into a bending position by the bending element of the cassette, and specific regions of this haptic part are thus no longer contacted by the plunger on its movement path. Moreover, by this configuration, the occurrence of undesired mechanic stresses of the lens such as they can occur for example upon mechanic force effects of the plunger on undesired locations of the lens, in particular on specific regions of the haptic part of the lens, can be avoided. In the context of the invention with bending of the haptic part of the lens an active bending with a element, the bending element, is meant. Passive bendings especially because of weight of the lens are not encompassed in the meaning of active bending. But in the embodiments the lens could also have such additional passive bendings, especially in an inclined position in the cassette.

Preferably, the bending element is disposed on a cover part of the cassette. With regard to the specific constructive configuration of the cassette and the shaping thereof, by the attachment, a particularly suitable configuration can be achieved, whereby the bending position can be adjusted very precisely on the one hand, and moreover, a highly functionally-reliable and mechanically stable principle is permitted. Moreover, by the attachment of the bending element to the cover part, a configuration very minimized in construction space is also ensured, yet the guide of movement of the plunger is not impaired.

Preferably, the bending element is pivotable about a horizontally formed pivot axis. By such a configuration, a particularly uniform force effect on the haptic part of the lens upon transfer from the rest-position into the bending position can be ensured such that undesired stresses or undesired lateral movements such as for example also torsions of the lens will not be able to occur in this connection.

Preferably, the bending element has at least one, in particular at least two bending tappets, which is or are disposed, respectively, for forming the bending position for contacting a top side of the haptic part, in particular a second section of the haptic part spaced from an optic part of the lens by a recess in the haptic part. This configuration of the cassette improves the uniform mechanic force effect of the bending element on the top side of the haptic part, whereby a uniform bending operation can be produced here too. Thereby, contact surfaces as large in area as possible are produced between the bending element and the top side of the haptic part such that undesired force peaks on a very small area can also be avoided in this respect. Not least, by this configuration, a contact of the bending element with the top side of the haptic part as large in area as possible can also be achieved. Thus, torsion of the haptic part about a longitudinal axis of the lens can also be avoided, and the entire haptic part can virtually be held in one plane, which is bent downwards.

Preferably, two bending tappets are provided on the bending element, which are disposed spaced from each other. In particular, between the two bending tappets, which are disposed on the same level viewed in longitudinal direction of the cassette, such a space is provided in a direction perpendicular to the longitudinal axis that an opening for feeding-through a plunger of an injector device is formed between the bending tappets. In multifunctional configuration, thus, with regard to a principle as compact as possible, a feed-through function for a plunger can also be provided besides the bending function. Thus, guide of the plunger can especially also be provided by the opening such that mechanically very precise guide is ensured also in this connection, and the plunger is guided to its intended point of contact with the lens.

Preferably, the bending position of the haptic part is formed in that a plunger of an injector device is displaceable in non-contacting manner to a front edge of the haptic part and past it during its complete movement path for advancing the lens out of the cassette, and an edge bounding a recess on the haptic part on the side of the optic part of the lens can be contacted by the plunger. By specific configuration of the lens and its specific position in the cassette as well as the specific movement path of the plunger, right on these conditions, a particularly reliable principle of action can be achieved, which prevents particularly critical positions of the lens, namely specific positions of the haptic part, from being able to be contacted by the plunger, and thus undesired deformations of or damages to these specific parts of the lens from being able to occur. However, it is ensured that mechanically stable parts of the lens, which are nevertheless not accessible for the plunger without adjusting the bending position due to the constructive configuration of the cassette and of the lens, are virtually only cleared for the contact with the plunger by the adjustment of the bending position.

Preferably, the bending element has an elevation protruding to the outside, which is formed for contact with a receiving frame upon introduction of the cassette into the receiving frame. Thus, a configuration of the bending element is produced, by which a mechanic principle of action is allowed, by which by a working step occurring anyway, namely inserting the cassette into the receiving frame, at the same time, then the relative movement of the bending element to the remaining components of the cassette is automatically effected. Therefore, also in this connection, it is not required that a user of the cassette and of the injector device has to explicitly manually operate himself the bending element, but this is effected automatically. Thereby, false operations of the bending element or operations too early or late in time can be avoided. By this mechanical principle of action, the optimum approach is performed virtually automatically at the correct time and also with regard to the correct bending path of the bending element such that undesired or false mechanic influences on the haptic part of the lens also cannot be effected in this connection.

Preferably, the bending element is formed such that it is automatically pivotable by the introduction into the receiving frame and the bending position of the haptic part is automatically formed in the final position of the cassette in the receiving frame. Here too, thus, an automatic mechanic principle of action can be provided, which automatically also achieves the precise bending position with regard to the introduction path of the cassette into the receiving frame and the final position reachable therein, such that here too, manual post-bending or adjustment or the like is no longer required.

Preferably, the elevation has a rounded edge contour on the side facing the receiving frame in the insertion direction. By such a configuration, a particularly smooth and continuous introduction can be allowed such that the pivoting movement of the bending element can also be effected very smoothly and uniformly, whereby in turn continuous and gentle transfer of the haptic part from the rest-position into the bending position can be achieved thereby too. Thereby, undesirably intense or jerky great mechanic force effects on the haptic part can be avoided.

Preferably, the bending element is formed integrally with the cassette, in particular a cover part. Preferably, the entire cassette is formed integrally and for example realized as a plastic part, in particular as an injection molding part. The bending element can also be formed on a base part of the cassette. The cassette includes the cover part and the base part, which are preferably connected through a film hinge and are movable relatively to each other.

In particular, it is provided that the lens is disposed in its rest-position in the cassette, and a position holding device is provided, which holds the lens in this rest-position. In particular, the position holding device is formed such that exactly fitting and positive abutment on the lens is not formed, but a specific tolerance of movement of the lens is possible in the rest-position. Especially, at least at one location of this position holding device, a shaping is formed, which allows a tolerance of movement of the lens of a maximum of 0.1 mm. By such a configuration of the position holding device, it can be ensured that the lens is not held rigidly and fixedly in the rest-position, and thereby, undesired friction damages to the exterior side of the lens can also be avoided. By the minimum tolerance of movement, exactly this flexibility of the lens can be maintained. In particular, the lens is wetted by a liquid when it is loaded in the cassette in the rest-position. Thus, due to the possible tolerance of movement, the lens can virtually "float" in the position holding device, but wherein the tolerance of movement is so small that undesirably great positional variations of the lens will not occur. In particular, the tolerance of movement is so small that it is negligible in value with regard to the fundamental orientation of the lens in the cassette in reference positions.

In particular, the position holding device is formed such that upon action of the bending element on a haptic part of the lens for producing the bending position, it virtually holds the remainder of the lens in the rest-position. Thus, by the bending element and the position holding device, only a specific haptic part is transferred into a bending position. The remainder of the lens remains unaffected by that and maintains its rest-position. In particular, that haptic part is transferred into a bending position, which faces a plunger of the injector device.

Preferably, the position holding device is formed such that the lens is positioned with its center-plane inclined with respect to a horizontal plane of the cassette in its rest-position in the state loaded in the cassette. By such an inclined arrangement of the lens in the rest-position, the matching between the adjustment of the bending position and the positioning and engagement of the bending element as well as the production of a movement path of the plunger as smooth-running and simple as possible can be accommodated in particularly advantageous manner.

It can also be provided that the center-plane of the lens is disposed horizontally and the rest-position of the lens thus extends in horizontal plane, wherein it can then be provided that the movement path of the plunger is also oriented in horizontal direction. In such a configuration, then, a relatively great bending of the haptic part has to be produced by the bending element, and therefore, the bending position is to be formed in a greater angle with respect to the center-plane of the lens. It can also be provided that in case of a horizontal orientation of the lens in its rest-position, the movement path of the plunger extends in a movement channel inclined thereto.

Preferably, the rest-position of the intraocular lens in the cassette is formed by the position holding device such that the center-plane and a longitudinal axis of a movement channel for the plunger, in particular a horizontal plane having the longitudinal axis, intersect in the region of an outer edge of an optic part of the intraocular lens, which has a recess in a haptic part, or a first section of a haptic part of the intraocular lens directly adjoining to an edge of the optic part.

In particular, the cassette includes a cover part and a base part movable relatively thereto, and the position holding device is formed by elements of the cover part and/or the base part, wherein, for this, the cover part and/or the base part have a shaping at least in certain regions on their surfaces facing the lens, which result in an inclined rest-position of the lens in the closed state of the cassette with the lens received.

In particular, the position holding device is formed such that a tolerance of movement is formed for the intraocular lens in the rest-position. This implies that presetting for the lens is provided by the position holding device, in which the lens can minimally move. Therefore, the position holding device is not an exactly fitting configuration positively abutting the on lens. Rather, the lens is virtually in a free state within the position holding device. However, the tolerances of movement are preferably 0.1 mm at the maximum at least at one location. This implies that at least at one location virtually a shaped shell is formed by the position holding device, which restricts the mobility of the lens to the top or the bottom to a maximum of 0.1 mm. Especially when a liquid is additionally loaded into the cassette, the lens can virtually float, however, it is held in the inclined position within the tolerances of movement by the position holding device. Excessive friction or excessive mechanic effects on the lens in the rest-position can thereby be avoided.

Preferably, the position holding device is formed such that the lens is disposed inclined to the bottom in the rest-position based on the end of the lens facing the entry region of the cassette. Thereby, the accessibility of the plunger to the lens can be achieved in particularly advantageous manner, and the advancement can thereby be achieved in simplest manner and with the lowest mechanical influences on the lens.

The haptic parts of the lens can be disposed angled with respect to the optic part. It can also be provided that the haptic parts and the optic parts are preferably disposed in one plane.

Preferably, a haptic part of the intraocular lens has a recess or a hole, by which a first section of the haptic part abutting the optic part of the lens, in particular a first section abutting a circumferential edge of the optic part, is formed. Moreover, by the recess, a second section of the haptic part spaced from the optic part is formed, which is relatively soft an deformable with regard to its configuration and which is therefore not contacted by the plunger on the peripheral side due to the specific rest-position in order to advance out the intraocular lens. Exactly this contact of the plunger with the outer edge of this second section of the haptic part is avoided by the present invention with respect to the cassette such that undesired deformations or undesired mechanic stresses and loads of this section of the haptic part can be avoided. In particular, the second section of the haptic part rests on the lay-on surface when the intraocular lens is disposed in its rest-position in the cassette.

By this configuration of the intraocular lens with these specific configurations of the haptic part and the inclined arrangement in the cassette, upon advancing-out and contacting the plunger, it can be achieved in the intraocular lens that this second section of the haptic part virtually remains nearly unaffected by the plunger and is in no case wiped on the peripheral side, but at most on the bottom or on the top, and the plunger slides along this bottom or top. An undesired mechanic deformation by contact with the plunger on the peripheral side at this second section of the haptic part can thus be avoided. By this position and configuration of the intraocular lens in the cassette, the haptic part can virtually be swept on the bottom or top with the plunger in particular with respect to its second section, and thus is no longer exposed to undesired mechanic loads in this respect.

Preferably, the intraocular lens is disposed in the cassette in its rest-position such that the second section of the haptic part is disposed above the movement path of the plunger. Thereby, the outer edge of the second section of the haptic part cannot be contacted by the plunger and, at best, contacting and sliding along the bottom of the second section of the haptic part can be effected by the plunger. This advantageously contributes to the avoidance of undesired deformations and mechanic loads upon advancing the intraocular lens out of the cassette.

Preferably, the rest-position is formed such that for advancing the lens out of the cassette, the plunger is disposed in non-contacting manner with the outer edge of the second section of the haptic part.
Preferably, the plunger engages the recess of the haptic part in certain regions for advancing the lens out of the cassette.
Preferably, in a first phase of advancing-out, the plunger is contacted with a bottom of the second section of the haptic part for lifting the lens. Thus, the plunger relatively gently slides along this bottom and lifts the intraocular lens upwards from its resting position on the lay-on surface of the bending element. By the plunger only engaging the bottom of this second section and sliding along it, undesired deformation of a second section of the haptic part is not caused. In a second phase of advancing-out, the plunger is preferably contacted with the first section of the haptic part and/or the optic part. After lifting, thus, furthermore, a position of contact of the plunger with the intraocular lens is reached, which is considerably more robust than the second section of the haptic part with respect to mechanic loads. Thereby, further advancing-out in this region of contact of the plunger with the intraocular lens can be ensured.

Preferably, the lens is disposed in its rest-position in the cassette such that the second section of the haptic part is disposed above the movement path of the plunger, in particular a bottom of the second section rests on the plunger after releasing the rest-position of the lens. Preferably, the lay-on surface of the bending element is respectively limited by an elevation at the front and at the back. In this respect, webs can be provided, between which the lay-on surface extends. By these elevations, the rest-position and the laid-on intraocular lens, in particular the second section of the haptic part of the intraocular lens, can be retained in particularly secure manner.

Preferably, the optic part of the intraocular lens is disposed in non-contacting manner with the bending element in the rest-position in the cassette. It proves particularly advantageous if the optic part of the lens is positioned substantially completely freely floating in the cassette in the rest-position. It can be provided that the optic part of the lens rests on a base part of the cassette with maximum 10 % of its area in the rest-position in the cassette and else is disposed in non-contacting and freely floating manner with the cassette. In this respect, thus, only contacting resting states of a first haptic part on the bending element on the one hand and of a further haptic part on a base part are provided, wherein at this place of the base part, the optic part formed adjacent thereto can still slightly rest on the base part also in direct manner.

Such a freely floating large-area arrangement of the intraocular lens allows a particularly target-oriented and gentle contact by means of the plunger and a particularly smooth-running lifting of the lens from its rest-position. Thereby, particularly gentle advancing-out formed with mechanic loads as low as possible can be effected.

Preferably, the angle of inclination between the horizontal plane and the lens, the center-plane of the lens, is formed between 5° and 40°, in particular 5° and 10°, especially 7° and 8°. They are particularly advantageous angular ranges with regard to a mechanically stable position formed with loads as low as possible on the one hand, and a particularly advantageous arrangement with regard to the simple and low-effort as well as low-load advancement of the lens out of the cassette on the other hand.

Preferably, the intraocular lens has two haptic parts, which are disposed on the edge on opposing sides of the optic part.

Preferably, the injection channel is formed as a hollow truncated cone, which thus tapers such that upon advancing the intraocular lens into this injection channel, automatic folding and thus rolling-up of the intraocular lens can be generated.

The invention also relates to a lens injector device having a cassette according to the invention or an advantageous development thereof. An intraocular lens is disposed in the cassette, and the injector device includes a movable plunger formed for advancing the intraocular lens out of the cassette and advancing it into an introduction channel of the lens injector device.

Preferably, the injector device has a receiving frame for the cassette. By introducing the cassette into the receiving frame, the bending element is automatically pivoted and the bending position of the haptic part of the lens is automatically formed.

Preferably, it is provided in the injector device that the cassette, in particular the position holding device of the cassette, is formed for receiving an intraocular lens, which has two, in particular only two, haptic parts formed on opposing positions of an optic part, wherein the rest-position of the lens in the cassette is formed such that a longitudinal axis of a movement channel in the cassette for the plunger extends through the two haptic parts or is an axis of symmetry of the lens through the haptic parts in one plane with the longitudinal axis in case of a position of the lens inclined with respect to the horizontal plane.

Advantageous implementations of the cassette according to the invention are to be considered as advantageous implementations of the injector device.

In a method according to the invention for advancing an intraocular lens out of a cassette, the intraocular lens is loaded into the cassette and the cassette is introduced into a receiving frame of a lens injector device. The lens is loaded into the cassette in a rest-position and a haptic part of the lens is transferred into a bending position by a bending element of the cassette before advancing-out. By this approach, undesired mechanic force effects by the plunger on specific parts of the lens, namely specific parts of the haptic part of the lens, can be avoided. Thereby, damages to the lens during the operation of advancing-out can be avoided.

Preferably, the bending position is automatically produced by the introduction of the cassette into the receiving space. Thereby, the mechanical effective connections and the different movements and operations of the individual components can be matched very exactly to each other with regard to the temporal sequence and functional cooperation. On the one hand, thereby, the user friendliness and the precision of the guide of movement are increased, and on the other hand, undesired force effects and undesired deformations of the lens are avoided.

Preferably, upon introduction of the cassette into the receiving frame, an elevation of the bending element is contacted with the receiving frame and thereby, the bending element is pressed into the cassette, wherein at least one bending element tappet, in particular two bending tappets spaced by an opening for pushing-through a plunger of the injector device, contacts or contact a top side of the haptic part, respectively, and the haptic part, in particular only the haptic part, is transferred into the bending position.

Preferably, a haptic part of the lens is formed with a continuous recess, by which an optic part of the lens is spaced from a second section of the haptic part, and the second section is transferred into the bending position by the bending element. Exactly this specific region of the haptic part is relatively soft and sensitive to intense mechanic force effects as they occur upon engagement and advancement of the lens by the plunger. By this specific bending of this second section, exactly such direct contact by the plunger can be avoided.

Preferably the bending position is formed such that a plunger is guided in non-contacting manner to a front lateral edge of the second section on its complete movement path for advancing the lens out of the cassette, and for advancing the lens, an edge of a first section of the haptic part and/or the optic part is contacted by the plunger. In particular with such specifically formed lenses having such a specific position in the cassette, it is particularly advantageous to guide the guide of movement of the plunger past this front lateral edge of the second section of the haptic part in non-contacting manner, wherein this is achieved in that this second section is bent out of the movement way of the plunger. Thereby, undesired mechanic force effects on this second section by the plunger are avoided and moreover, in addition, it can be achieved that mechanically stable regions of the lens insensitive to the force effects of the plunger upon advancing-out, can be cleared and directly contacted by the plunger.

The recess in the haptic part of the lens can also be formed such that there is no first section of the haptic part, but the recess is partially directly bounded by the edge of the optic part. In the bending position of the haptic part, the plunger then engages the optic part, in particular the edge thereof.

Preferably, the intraocular lens is positioned inclined with respect to a horizontal plane of the cassette in a rest-position of the cassette, in particular a center-plane of the intraocular lens is disposed in an angle with respect to the horizontal plane such that the lens is disposed inclined to the bottom based on the end of the lens facing the entry region of the cassette.

Advantageous implementations of the cassette according to the invention and of the lens injector device according to the invention are to be considered as advantageous implementations of the method according to the invention.

Further features of the invention appear from the claims, the figures and the description of figures. The features and feature combinations mentioned above in the description as well as the features and feature combinations mentioned in the description of figures alone as well as the features and feature combinations only shown in the figures are usable not only in the respectively indicated combination, but also in other combinations or alone without departing from the scope of the invention. Explained embodiments can also be combined in individual features to new embodiments.

### Brief description of the drawings

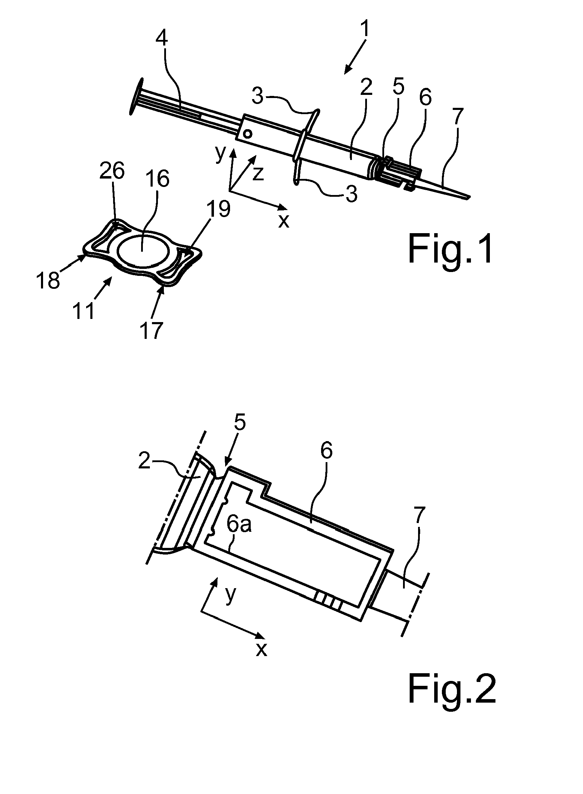

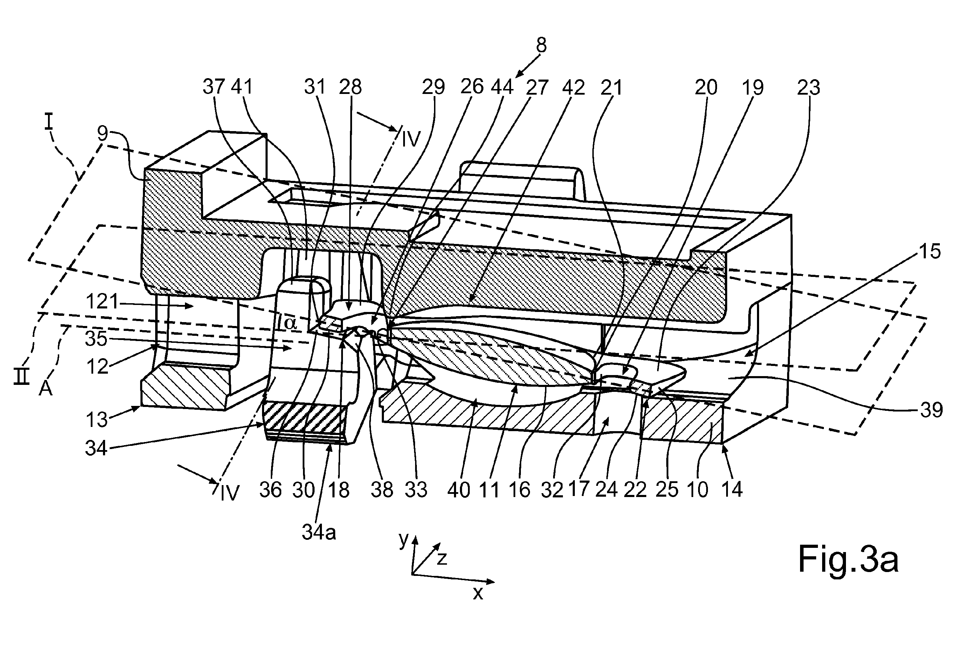

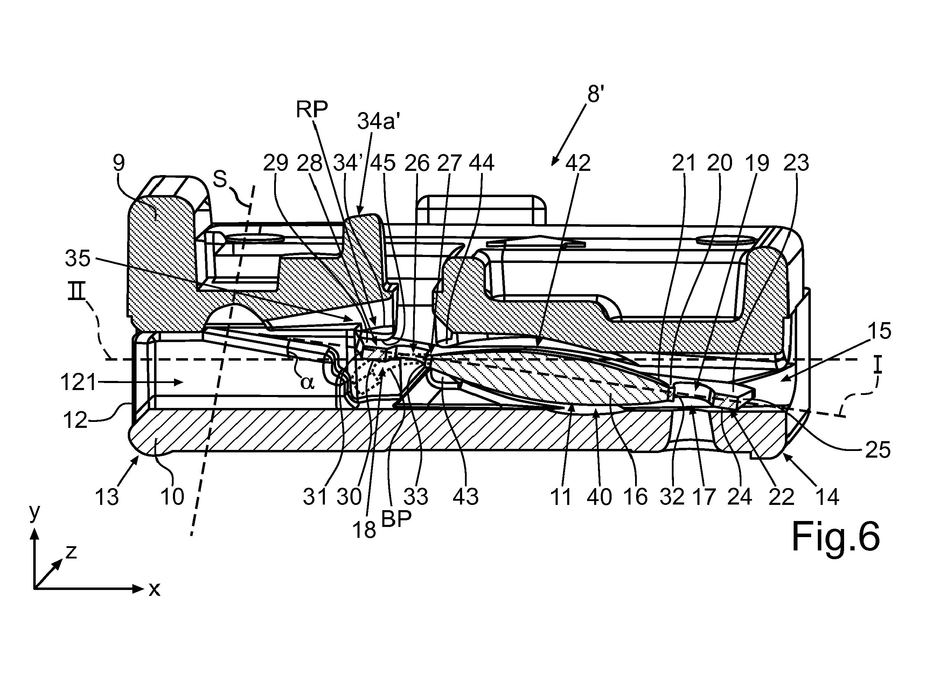

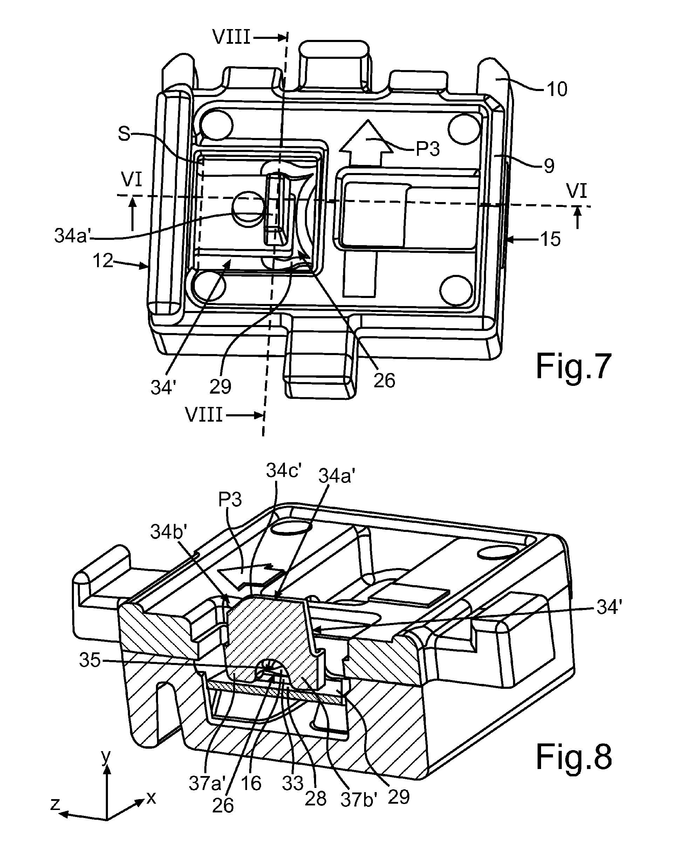

Embodiments of the invention are explained in more detail below based on schematic drawings. There show:
a perspective representation of an embodiment of a lens injector device according to the invention;
an enlarged representation of a partial section of the lens injector device according to fig. 1;
a perspective sectional representation of an embodiment of a cassette according to the invention with an intraocular lens disposed in the rest-position;
a section of the representation according to fig. 3a and in schematic representation additionally the bending position of the haptic part of the intraocular lens;
another sectional representation through the cassette according to fig. 3a, in which the rest-position and the bending position of the haptic part of the intraocular lens are schematically shown;
5a and 5b schematic simplified representations of different rest-positions and bending positions of the intraocular lens as well as an exemplary orientation of a plunger of the injector device;
a sectional representation through a further embodiment of a cassette according to the invention with a rest-position and a schematically shown bending position of the haptic part of the intraocular lens;
a perspective top view of the cassette according to fig. 6;
a further perspective sectional representation of the cassette with the intraocular lens according to fig. 6 and 7; and
a further schematic representation of the intraocular lens in the rest position and the bending position as well as an exemplary orientation of the plunger.

In the figures, similar or functionally similar elements are provided with the same reference characters.

In fig. 1 and fig. 2, in a perspective representation, a lens injector device 1 is shown, which is formed for introducing an intraocular lens into an eye. The injector device 1 includes a tubular hollow body 2, on which two gripping members 3 and 4 are disposed at the outside. Moreover, the injector device 1 includes a plunger 4, which is displaceable in the tubular hollow body 2 in the direction of the x-axis. On a front end 5 of the tubular hollow body 2, an insertion region or receiving region 6, respectively, is formed, which is formed as a receiving frame. Following this receiving region 6, which is formed for receiving a cassette with an intraocular lens, a tapering hollow-shaped introduction part 7 is disposed. The tubular hollow body 2 has an opening at the end 5, through which the plunger 4 can slide to advance out the lens in the cassette, which is then disposed in the receiving region 6, and to advance it into the introduction part 7. Due to the tapering configuration of the introduction part 7, the intraocular lens is automatically rolled up upon advancing into this introduction part 7.

In fig. 2, the receiving frame 6 for the cassette 8 is shown, wherein it has an edge 6a. The receiving frame 6 extends in z-direction and thus perpendicularly to the figure plane over a certain depth such that the receiving frame 6 virtually is an angular hollow body. For example, this is apparent in fig. 1.

The cassette 8 includes a cover part 9 and a base part 10. The cover part 9 and the base part 10 are movable relatively to each other and are correspondingly openable and closeable for inserting an intraocular lens 11 into the cassette 8.

In fig. 3a, a sectional representation through the cassette 8 is shown, wherein the sectional plane extends in the x-y-plane. The cassette 8 is shown in the closed state in fig. 3a and the intraocular lens 11 is disposed in its rest-position.

The cassette 8 has an entry region 12, which follows the end 5 of the tubular hollow body 2. The plunger 4 can be advanced into the interior of the cassette 8 through the entry region 12. The entry region 12 is formed at the end 13 of the cassette 8 facing the tubular hollow body 2. At the opposing end 14 of the cassette 8 facing the introduction part 7, an exit region 15 is formed. The intraocular lens 11 can be advanced out of the cassette 8 by means of the plunger 4 through this exit region 15 and be advanced into the introduction part 7 of the injector device 1.

Thus, the plunger 4 is moved in the x-direction to advance the intraocular lens 11 out of the cassette 8.

Subsequent to the entry region 12, the cassette 8 has a movement channel 121, in which the plunger 4 is guided to access the lens 11. The movement channel 121 has a longitudinal axis A, which extends horizontally and thus extends in x-direction. Correspondingly, the plunger 4 is guided horizontally. The longitudinal axis A extending in the horizontal plane II and a center-plane I of the lens 11 are disposed inclined to each other, wherein an angle α is formed.

In its shown rest-position in the cassette 8, the intraocular lens 11 is positioned inclined to the bottom. The intraocular lens 11 has a central optic part 16, on which in the embodiment a haptic part 17 and 18 are respectively disposed at opposing ends. The intraocular lens 11 only has these two haptic parts 17 and 18, wherein they are disposed at opposing ends of the optic part 16 considered in x-direction according to the representation. In the embodiment, the haptic parts 17 and 18 are not disposed angled with respect to the optic part 16, but formed in one plane with the optic part 16.

A recess 19 is formed in the first haptic part 17. A first section 20 of the haptic part 17 is formed by this recess 19. This first section 20 is relatively thin and immediately abuts the circumferential edge 21 of the optic part 16. Moreover, a second section 22 of the haptic part 17 is formed by the recess 19. It is formed spaced from the optic part 16. The first haptic part 17 has a top 23 and a bottom 24. Moreover, a lateral edge 25 is formed, which faces the exit region 15 in the rest-position.

In corresponding manner, the further haptic part 18 is formed. There too, a recess 26, which is continuous, is formed. A first section 27 of the haptic part 18 is formed by this recess 26, which again is relatively thin and immediately abuts the circumferential edge 21 of the optic part 16. A second section 28 of the haptic part 18 spaced from the optic part 16 is formed by the recess 26. Here too, the further haptic part 18 has a top 29 and a bottom 30. Moreover, a lateral edge 31 connecting the top 29 and the bottom 30 is formed.

Moreover, a lateral edge 32 at the first section 20 and a lateral edge 33 at the first section 27 are formed by the recesses 19 and 26.

The inclined position of the intraocular lens 11 in its rest-position is **characterized in that** the end of the intraocular lens facing the entry region 12 is disposed higher in y-direction than the end of the intraocular lens 11 facing the exit region 15.

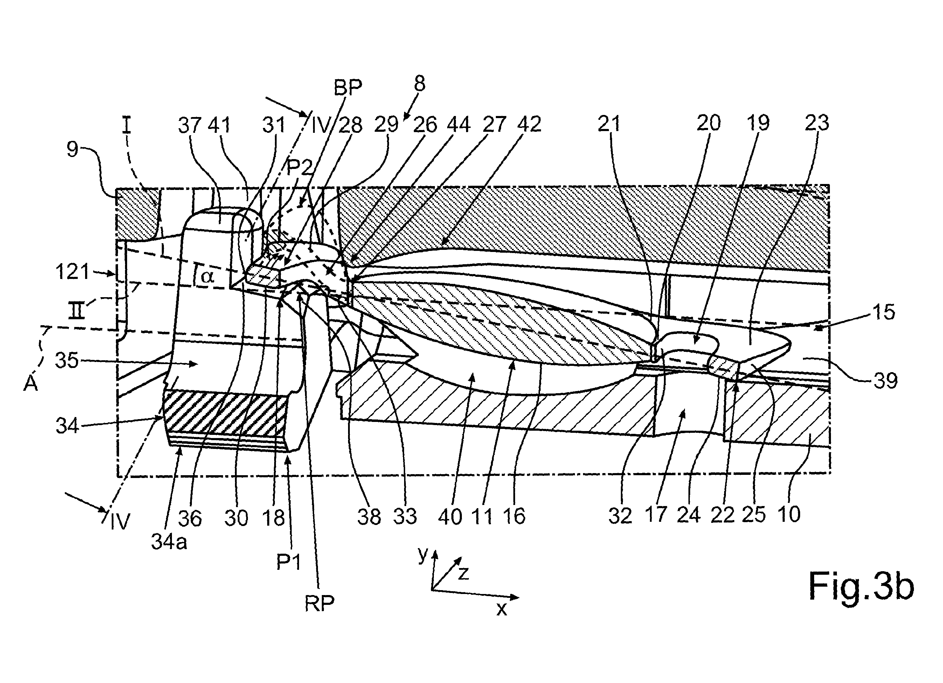

In particular, the rest-position of the intraocular lens 11 is formed such that a center-plane I of the intraocular lens 11 is disposed in an angle α with respect to a horizontal plane II. The horizontal plane II extends in the x-z-plane, wherein the moving direction of the plunger 4 is effected in the horizontal plane II or parallel thereto. The center-plane I of the intraocular lens II extends through the two haptic parts 17 and 18 as well as through the optic part 16 as it is illustrated in fig. 3a and 3b. The angle α preferably is between 5° and 40°, in particular between 5° and 10°, especially 7,5°.

In the shown implementation, the base part 10 includes a bending element 34, which has a guide-through opening 34 for the plunger 4. Moreover, a lay-on surface 36 is provided, on which the end of the intraocular lens 11 facing the entry region 12 rests. In particular, the haptic part 18 and only the second section 28 thereof rests on this lay-on surface 36 with its bottom 30.

Considered in x-direction, the lay-on surface 36 is limited by a first elevation 37 and a second elevation 38, which extend upwards in y-direction. The second elevation 38 is lower than the first elevation 37. In the shown rest-position of the intraocular lens 11, thus, it is also retained by the bending element 34 with respect to undesired displacements in the x-direction.

Moreover, the first haptic part 17 rests on a bottom surface 39 of the base part 10 with its bottom 24. Moreover, the base part 10 includes a well region 40, which is formed approximately on the level of the optic part 16 considered in x-direction in the rest-position of the intraocular lens 11. In the rest-position, moreover, the intraocular lens 11 is positioned such that the optic part 16 is disposed in non-contacting manner with the base part 10 and the cover part 9. At best, it can be provided that the optic part 16 rests on the bottom surface 39 with a very small surface. Thus, the optic part 16 is virtually disposed freely floating in the rest-position within the cassette 8.

In particular, the lens 11 is held in the inclined rest-position by a position holding device 42. The position holding device 42 is formed by elements of the cover part 9 and the base part 10. In particular, the shapings of the cover part 9 and of the base part 10 are formed in the region of the lens 11 such that the surfaces facing the lens are adapted to the shape of the lens 11 in certain regions. However, it is essential that this shaping is formed with a tolerance of movement for the lens 11. This implies that the lens 11 is not form-closed contacted by the cover part and the base part in positive manner, but that a shaped shell is virtually formed, which allows a small movement of the lens 11 in all three spatial directions. Since, in particular, a bit of liquid also wets the lens 11, the lens is "floating" in a free state in the cassette 8, and yet is held in the inclined position. The tolerance of movement is smaller than or equal to 0.1 mm at least at one location, such that the free mobility of the lens 11 is greatly kept within limits since the inclined position is only minimally variable. In the embodiment, the location with the minimum tolerance of movement is in fig. 3a and 3b in the region of the recess 26, between an elevation 38 on the bending element 34 and a convex element 44 on the cover part 9.

Preferably, the rest-position of the intraocular lens 11 is provided such that the center-plane I and the horizontal plane II intersect in the region of the optic part 16 and the first section 27 of the haptic part 18. This line of intersection between the two planes I and II is therefore locally localized to a very small region, which extends in the region of the lateral edge 33 of the first section 27 and due to the low thickness of the first section 27 in x-direction therefore also in the region of the lateral edge 21 of the optic part 16.

This positioning is particularly advantageous with regard to the contact of the plunger 4 with the intraocular lens 11 and the further procedure of advancement out of the cassette 8.

The cassette 8 has a receiving space 41 in the cover part 9, into which the lens 11, in particular the second haptic part 18, can be lifted when the lens 11 is advanced out.

In the rest-position, the second haptic part 18 is disposed above the movement path of the plunger 4. Considered in x-direction, the two haptic parts 17 and 18 are disposed one behind the other.

In the representation according to fig. 3a, the rest-position of the lens 11 is shown. In this connection, the lens 11 is formed with haptic parts 17 and 18, which do not have any fundamental inclination and angulation with respect to the center-plane I. Principally, such a lens can also be loaded into the cassette 8, in which the haptic parts 17 and 18 are formed angled with respect to the center-plane I. In the rest-position RP shown in fig. 3a, the cassette 8 is not yet disposed in the receiving frame 6. Based on this situation, then, the cassette 8 is advanced into the receiving frame 6 with the intraocular lens 11 disposed therein in the rest-position RP, wherein this is effected in z-direction. Therein, the entry region 12 faces the hollow body 2. Upon introducing the cassette 8 into the receiving frame 6, the bending element 34 contacts the edge 6a with its elevation 34a extending to the outside. Due to the protrusion, upon further advancement of the cassette 8 into the receiving frame 6, the bending element 34 is pressed in y-direction. Thereby, the haptic part 18 and in particular the second section 28 are automatically and continuously bent out of the rest-position RP and taken into a bending position BP, as it is shown in fig. 3b, upon further advancement of the cassette 8 into the receiving frame 6. This bending position BP is held by the bending element 34 when the cassette 8 has reached its final position in the receiving frame 6. As it is apparent from the representation in fig. 3b, this second section 28 is correspondingly bent upwards according to the arrow representation P2, if the force for impressing acts on the elevation 34a in the direction of the arrow P1.

By this transfer of the second section 28 from the rest-position RP into the bending position BP, it is achieved that in the following, a plunger 4 of the injector device 1 can be advanced into the movement channel 121 and moves through the opening 35 and can be guided in non-contacting manner to the front edge 31 of the second section 28 on its movement path. Thus, the plunger 4 is guided into the recess 26 below the second section 28 and then the plunger 4 directly engages with the first section 27 and/or the optic part 16 due to the position of the lens 11. In the bending operation by the bending element 34, only the haptic part 18, in particular the second section 28, is taken into the bending position BP, wherein the remainder of the lens 11 substantially remains unchanged in the rest-position RP due to the position holding device 42.

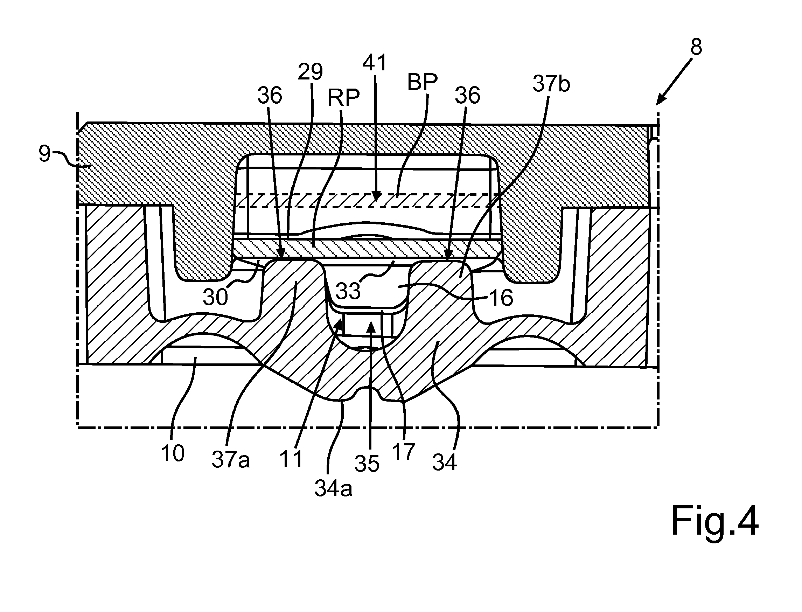

In fig. 4, a further sectional representation along the sectional line IV-IV in fig. 3a and 3b is shown. In this view in x-direction, the cassette 8 is virtually shown from the plunger 4. The inclined arrangement of the intraocular lens 11 formed towards the exit region 15 is recognizable.

Moreover, by the rest-position of the intraocular lens 11, it is also achieved that the plunger 4, upon contacting the intraocular lens 11, either contacts the second section 28 of the haptic part 18 or at best contacts the bottom 30 and wipes along it. The front lateral edge 31 of the second section 28 of the haptic part 18 is no longer contacted by the plunger 4 and therefore, a mechanic force in x-direction is no longer exerted in this respect. By this configuration, undesired force effect by the plunger 4 on this second section 28 of the haptic part 18 can be avoided. The lateral edge 31 is disposed free from bending in the rest-position of the lens 11 in the cassette 8 and particularly also during the entire approaching distance of the plunger 4 until reaching the advancement contact position on the lens 11. This implies that bending of the haptic part 18 facing the plunger 4, in particular of the second section 28, by an element of the cassette 8, which also is not associated with the plunger 4, is not performed in order to be able to avoid a contact of the plunger 4 with the lateral edge 31. Thereby, undesired deformations upon advancing the intraocular lens 11 out of the cassette 8 can be avoided. Exactly the haptic parts, in particular the exterior second sections 28 and 22 thereof, which are soft and sensitive with regard to mechanic loads, can therefore be relieved from undesired mechanic stresses and loads.

In the further sectional view shown in fig. 4, both the rest position RP is shown and the bending position BP bent upwards is shown dashed. Moreover, the configuration of the bending element 34 is apparent. In the shown implementation, it has 2 bending tappets 37a and 37b formed spaced from each other in z-direction. The distance in z-direction is dimensioned such that the plunger 4 can be advanced through the opening 35 and moreover is guided therein. Moreover, the two bending tappets 37a and 37b are formed the same in height and shaping. Thereby, contact of the area or bottom 30 of the section 28 as large in area and as uniform as possible by the supporting surface 36 of the bending tappets 37a and 37b can be allowed.

In fig. 4 like in fig. 3b, the position of the bending element 34 displaced to the top in y-direction is not shown in order to be able to ensure the clarity in the figures. Only the exemplarily achieved final end position of the bending position BP of the second section 28 is schematically represented.

According to the representations in fig. 3a, 3b and fig. 4, furthermore, the advancement of the intraocular lens 11 out of the cassette 8 with the plunger 4 is explained. If the cassette 8 is inserted in the receiving region 6, the plunger 4 can be introduced into the cassette 8 through the entry region 12 of the cassette 8. Therein, it then gets to the intraocular lens 11 through the movement channel 121 and the guide-through opening 35 in the bending element 34. Due to the inclined position and the raised positioning by means of the bending element 34, the plunger 4 virtually engages below the intraocular lens 11. The position of the intraocular lens 11 can be formed by the position holding device 42 such that the plunger 4 slides in non-contacting manner past the second section 28 of the haptic part 18 and only directly engages the first section 27, in particular the edge 33, of the haptic part 18 and/or the optic part 16 of the intraocular lens 11. By the further movement of the plunger 4 in x-direction, then, the intraocular lens 11 is moved from its rest-position and advanced in x-direction to the exit region 15. Therein, the second section 28 of the haptic part 18 is released from the lay-on surface 16 and then rests on the plunger 4.

It can also be provided that the position of the intraocular lens 11 in the cassette 8 is formed by the position holding device 42 such that upon advancement into the cassette 8, the plunger 4 will also again not engage the lateral edge 31, but only contacts the bottom 30 of the second section 28 and slides along it in x-direction. Thereby, the intraocular lens 11 is first lifted in y-direction. Preferably, the lifting is such that the second section 28 is on the level of or higher than the extension of the elevation 33 in y-direction. In the further advancement of the plunger 4 in x-direction, then, the front side of the plunger 4 is contacted with the first section 27, for example with the edge 33, and/or directly contacted with the optic part 16 and advanced in the direction of the exit region 15.

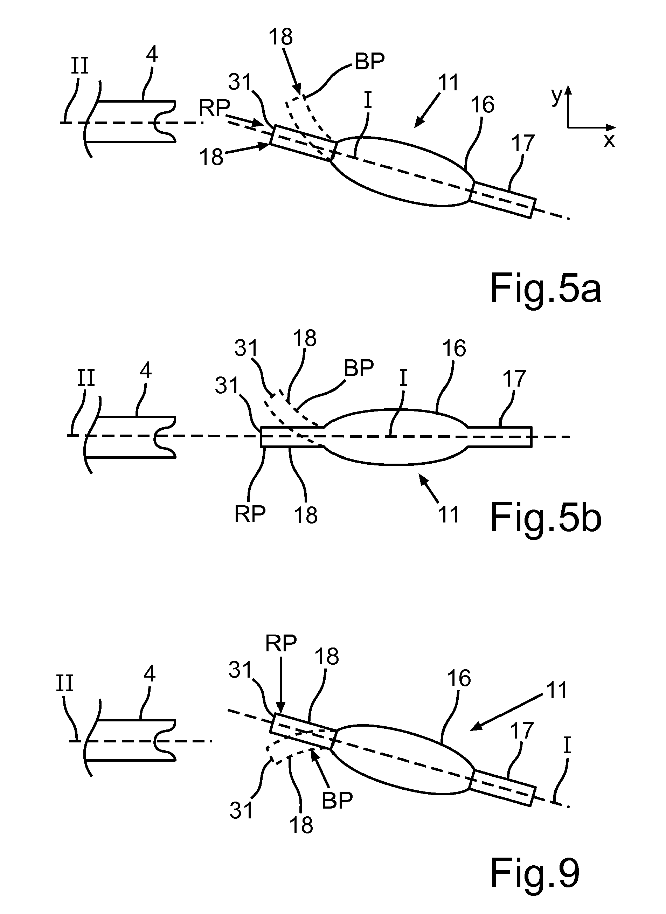

In fig. 5a and 5b, in very simplified and schematic representation, side views of the lens 11 in different orientations are shown. Thus, in fig. 5a, a rest-position RP of the lens 11 is shown, which is disposed inclined with respect to the horizontal extending in x-direction. This corresponds to the representation according to fig. 3a and 3b. It is apparent that the lens 11 has haptic parts 17 and 18 in the shown embodiment, which extend in the center-plane I and thus are not formed angled with respect to the optic part 16. Basically, a lens can also be provided and inserted into the cassette 8, which has haptic parts 17 and 18 angled with respect to the optic part 16 in the basic configuration.

In the representation according to fig. 5a, moreover, the bending position BP is shown, wherein, as already explained, only the haptic part 18, in particular the second section 28, is bent upwards. Thereby, the movement path for the plunger 4 moving in horizontal direction, to the optic part 16 and/or to the first section 27 can be cleared. In the rest-position RP, the plunger 4 would contact the front lateral edge 31 on its movement path. By transferring the haptic part 18 into the bending position BP, non-contacting movement of the plunger 4 to this lateral edge 31 can be ensured.

In fig. 5b, also in very simplified and schematic representation, a further implementation is shown, wherein the intraocular lens 11 is positioned with its center-plane I in horizontal arrangement in its rest-position RP. Analog to this, the plunger 4 is also disposed with its longitudinal axis in the horizontal plane II and the direction of movement is in x-direction. Also in this configuration, based on the rest-position RP, then the bending position BP is adjusted by bending the haptic part 18 facing the plunger 4 to the top. Corresponding explanations to fig. 5a are furthermore also analog for fig. 5b.

At this point, it is to be emphasized that the implementations in fig. 3a, 3b and fig. 4 are exemplary. An inverse configuration can also be provided, in which the bending element 34 is disposed as a bending element 34' on the cover part 9, as it is shown in fig. 6 according to the sectional representation there. The location with the minimum tolerance of movement for the lens 11 in the position holding device 42 is formed in the region between the elements 43 on the base part 10 and the overlying cover part region. Here too, depending on the amount of liquid around the lens 11, either a lay-on surface of a raised element 43 of the base part 10 or an opposing element 44 formed at the location on the cover part 9, and/or a contact surface 45 of a bending element 34' can serve as a stop for the lens 11.

Moreover, in fig. 6, a pivot axis S is schematically drawn, which extends in z-direction. The bending element 34' can be pivoted downwards about this pivot axis S. Thereby, upon force effect from the top, the bending element 34' can be pressed into the cassette 8. Therein, also in this embodiment, the elevation 34a' protruding outwards from the cassette 8 is contacted upon advancement into the receiving frame 6, and thereby the bending element 34' is pressed inwards. Thereby, the bending tappets 37a' and 37b' (fig. 8) facing the haptic part 18 of the lens 11, in particular the second section 28, are pressed downwards, and thereby the top side 29 is automatically contacted and the section 28 of the haptic part 18 is bent downwards and taken into the bending position BP. In particular, thus, the front lateral edge 31 of the second section 28 is bent out of the movement path of the plunger 4 such that along the movement path of the plunger 4 it can be guided in non-contacting manner to this lateral edge 31 and does not contact it. In particular, by this transfer of the second section 28 from the rest-position RP into the bending position BP, it is achieved that the plunger 4 virtually can also slide into the recess 26 and can attain a mechanic contact of the edge 33 and thus of the first section 27 and/or the optic part 16.

In fig. 9, a very simplified schematic side view for the implementation in fig. 6 is shown thereto, wherein both the rest-position RP and the bending position BP of the haptic part 18 facing the plunger 4 are shown.

Serving for clarity, in fig. 6, the bending position BP is only shown dashed and very simplified.

In the completely loaded state of the cassette 8' in the receiving frame 6, the final bending position BP and thus also the final position in this respect is also automatically reached. Then, it is also held automatically by the bending element 34'. This is effected until the plunger 4 engages the first section 27 of the haptic part 18 and/or on the optic part 16 and advances the lens 11 out in x-direction.

In fig. 7, a top view of the cassette 8' according to fig. 6 is shown. In this respect, on the cover 9, the direction P3 is also symbolically indicated, in which the cassette 8' has to be introduced into the receiving frame 6. In fig. 7, a sectional line VI-VI is represented, which shows the sectional line for the sectional representation in fig. 6. Moreover, a sectional line VIII-VIII is shown, wherein the associated sectional representation is shown in fig. 8.

In this sectional representation in fig. 8, the bending element 34' is shown. Therein, the bending tappets 37a' and 37b' formed spaced from each other in z-direction and of equal shape are shown. An opening 35 for the plunger 4 is formed between these bending tappets 37a' and 37b'. The bending tappets 37a' and 37b' engage the top side 29 of the second section 28 virtually approaching from the top to transfer it from the rest-position RP into the bending position BP. Moreover, it is apparent in fig. 8 that the elevation 34a' has an edge contour 34c' rounded downwards on the side 34b' facing upon introduction into the receiving frame 6. Thereby, particularly gentle contact and introduction into the receiving frame 6 can be effected. Furthermore, thereby, particularly jerk-free and continuous downwards pivoting of the bending element 34' can also be ensured such that undesired jerky force effects on the haptic part 18, in particular the second section 28, upon pressing downwards also will not occur.

## Claims

1. Cassette for receiving an intraocular lens (11), wherein the cassette (8, 8') is formed for inserting into a lens injector device (1) and has an entry region (12) for a plunger (4) of the injector device (1) and an exit region (15) for the lens (11), **characterized in that** the cassette (8, 8') has a bending element (34, 34'), which is disposed movable on the cassette (8, 8') and which is formed for transferring a haptic part (17, 18) of the intraocular lens (11) loaded in the cassette (8, 8') from a rest-position (RP) into a bending position (BP).

2. Cassette according to claim 1, **characterized in that** the bending element (34, 34') is disposed on a cover part (9) of the cassette (8, 8').

3. Cassette according to claim 1 or 2, **characterized in that** the bending element (34, 34') is pivotable about a horizontally formed pivot axis (S).

4. Cassette according to anyone of the preceding claims, **characterized in that** the bending element (34, 34') has at least one, in particular at least two, bending tappets (37a', 37b'), which are disposed for forming the bending position (BP) for contacting a top side (29) of the haptic part (17, 18), in particular a second section (22, 28) of the haptic part (17, 18) spaced from an optic part (16) of the lens (11) by a recess (19, 26) in the haptic part (17, 18), especially an opening (35) for feeding-through a plunger (4) of an injector device (1) is formed between the two bending tappets (37a', 37b').

5. Cassette according to anyone of the preceding claims, **characterized in that** the bending position (BP) of the haptic part (17, 18) is formed **in that** a plunger (4) of an injector device (1) is displaceable in non-contacting manner to a front edge (31) of the haptic part (17, 18) and past it in non-contacting manner during its entire movement path for advancing the lens (11) out of the cassette (8, 8'), and an edge (33) bounding a recess (19, 26) in the haptic part (17, 18) on the side of the optic part (16) of the lens (11) can be contacted by the plunger (4).

6. Cassette according to anyone of the preceding claims, **characterized in that** the bending element (34, 34') has an elevation (34a, 34a') protruding to the outside, which is formed for contact with a receiving frame (6) upon introduction of the cassette (8, 8') into the receiving frame (6), especially that the bending element (34, 34') is formed such that it is automatically pivotable by the introduction into the receiving frame (6) and the bending position (BP) of the haptic part (17, 18) is automatically formed in the final position of the cassette (8, 8') in the receiving frame (6), and/or that the elevation (34a, 34a') has a rounded edge contour (34c') on the side (34b') facing the receiving frame (6) in the insertion direction (P3).

7. Cassette according to anyone of the preceding claims, **characterized in that** the bending element (34, 34') is formed integrally with the cassette (8, 8'), in particular a cover part (9).

8. Cassette according to anyone of the preceding claims, **characterized in that** the cassette (8, 8') has a position holding device (42), by which the lens (11) is positioned with its center-plane (I) inclined with respect to a horizontal plane (II) of the cassette (8, 8') in its rest-position (RP) in the state loaded in the cassette (8, 8'), especially that the rest-position (RP) of the intraocular lens (11) in the cassette (8, 8') is formed by the position holding device (42) such that the center-plane (I) and a longitudinal axis (A) of a movement channel (121) of the plunger (4), in particular a horizontal plane (II) having the longitudinal axis (A), intersect in the region of an outer edge (21) of an optic part (16) of the intraocular lens (11), which has a recess (19, 26) in a haptic part (17, 18), or a first section (20, 27) of a haptic part (17, 18) of the intraocular lens (11) directly adjoining to an edge (21) of the optic part (16).

9. Lens injector device (1) including a cassette (8, 8') according to any one of claims 1 to 12, in which an intraocular lens (11) is disposed, and having a movable plunger (4) formed for advancing the intraocular lens (11) out of the cassette (8, 8') and for advancing it into an introduction channel (7) of the lens injector device (1).

10. Injector device according to claim 9, in which a receiving frame (6) for the cassette (8, 8') is formed, and by the introduction of the cassette (8, 8') into the receiving frame (6), the bending element (34, 34') is automatically pivoted and the bending position (BP) of the haptic part (17, 18) is automatically formed, and/or in which the cassette (8, 8'), in particular the position holding device (42), is formed for receiving an intraocular lens (11), which has two haptic parts (17, 18) formed on opposing positions of an optic part (16), wherein the rest-position (RP) of the lens (11) in the cassette (8, 8') is formed such that a longitudinal axis (A) of a movement channel (121) of the plunger (4) extends through the two haptic parts (17, 18) or is an axis of symmetry of the lens (11) through the haptic parts (17, 18) in one plane with the longitudinal axis (A) in case of a position of the lens (11) inclined with respect to the horizontal plane (II).

11. Method for advancing an intraocular lens (11) out of a cassette (8, 8'), in which the intraocular lens (11) is loaded into the cassette (8, 8') and the cassette (8, 8') is introduced into a receiving frame (6) of a lens injector device (11), **characterized in that** the lens (11) is loaded into the cassette (8) in a rest-position (RP) and a haptic part (17, 18) of the lens (11) is transferred into a bending position (BP) by a bending element (34, 34') of the cassette (8, 8') before advancing-out.

12. Method according to claim 11, **characterized in that** the bending position (BP) is automatically produced by the introduction of the cassette (8, 8') into the receiving frame (6), especially that upon introduction of the cassette (8, 8') into the receiving frame (6), an elevation (34a, 34a') of the bending element (34, 34') is contacted with the receiving frame (6), and thereby, the bending element (34, 34') is pressed into the cassette (8, 8'), and by at least one bending tappet (37a', 37b'), in particular two bending tappets (37a', 37b') spaced by an opening (35) for pushing-through a plunger (4) of the injector device (1), a top side (29) of the haptic part (17, 18) is contacted and the haptic part (17, 18), in particular only the haptic part (17, 18), is transferred into the bending position (BP).

13. Method according to any one of claims 11 or 12, **characterized in that** a haptic part (17, 18) of the lens (11) has a recess (19, 26), by which an optic part (16) of the lens (11) is spaced from a second section (22, 28) of the haptic part (17, 18), and the second section (22, 28) is transferred into the bending position (BP) by the bending element (34, 34'), especially that the bending position (BP) is formed such that a plunger (4) is guided in non-contacting manner to a front lateral edge (31) of the second section (28) on its entire movement path for advancing the lens (11) out of the cassette (8, 8') and contacts an edge (33) of a first section (20, 27) of the haptic part (17, 18) and/or the optic part (16) for advancing the lens (11) out.

14. Method according to anyone of the preceding claim 11 to 13, **characterized in that** the intraocular lens (11) has two haptic parts (17, 18), which are formed on opposing positions of an optic part (16), wherein the lens (11) is loaded into the cassette (8, 8') in its rest-position (RP) such that a longitudinal axis (A) of a movement channel (121) of the plunger (4) extends through the two haptic parts (17, 18) or an axis of symmetry of the lens (11) extends through the haptic parts (17, 18) in one plane with the longitudinal axis (A) in case of a position of the lens (11) inclined with respect to the horizontal plane (II).

15. Method according to any one of claims 11 to 14, **characterized in that** the intraocular lens (11) is positioned inclined with respect to a horizontal plane (II) of the cassette (8) in a rest-position (RP) in the cassette (8, 8'), in particular a center-plane (I) of the intraocular lens (11) is disposed in an angle (OC) with respect to the horizontal plane (II) such that the lens (11) is disposed inclined to the bottom based on the end of the lens (11) facing the entry region (12) of the cassette (8, 8').
